# EUROPEAN PATENT SPECIFICATION

(11) **EP 2 870 241 B1**
(45) Date of publication and mention of the grant of the patent: **14.03.2018**
(21) Application number: 14703938.2
(22) Date of filing: 14.01.2014
(51) Int. Cl.: C12Q 1/68, C07K 16/00, C12N 9/78

(54) **A COMPOSITION SHOWING ACTIVATION-INDUCED CYTIDINE DEAMINASE (AID) ACTIVITY, A METHOD OF PREPARATION OF SUCH COMPOSITION IN A BACTERIAL SYSTEM AND USE OF THE COMPOSITION IN THE ANALYSES OF DNA/RNA AMINATION/DEAMINATION AND/OR METHYLATION/DEMETHYLATION**
ZUSAMMENSETZUNG MIT AKTIVIERUNGSINDUZIERTER CYTIDINDEAMINASE (AID)-AKTIVITÄT, VERFAHREN ZUR HERSTELLUNG SOLCH EINER ZUSAMMENSETZUNG IN EINEM BAKTERIELLEN SYSTEM UND VERWENDUNG DER ZUSAMMENSETZUNG IN DER ANALYSE VON DNA/RNA-AMINIERUNG/-DESAMINIERUNG UND/ODER -METHYLIERUNG/-DEMETHYLIERUNG
UNE COMPOSITION PRÉSENTANT L'ACTIVITÉ CYTIDINE DÉSAMINASE INDUITE PAR L'ACTIVATION (AID), UN PROCÉDÉ DE PRÉPARATION D'UNE TELLE COMPOSITION DANS UN SYSTÈME BACTÉRIEN ET L'UTILISATION DE LA COMPOSITION DANS LES ANALYSES D'AMINATION/DÉSAMINATION ET/OU MÉTHYLATION/DÉMÉTHYLATION D'ADN/ARN

(30) Priority: 14.01.2013 PL 40244013
(43) Date of publication of application: 13.05.2015
(73) Proprietor: Instytut Chemii Bioorganicznej PAN, 61-704 Poznan (PL)
(72) Inventor: BUDZKO, Lucyna, 61-049 Poznan (PL); JACKOWIAK, Paulina, 61-615 Poznan (PL); FIGLEROWICZ, Marek, 62-023 Borówiec (PL)
(74) Representative: Witek, Rafal
(86) International application number: PCT/PL2014/000003
(87) International publication number: WO 2014/109656

(56) References cited:
- WO-A1-2010/113039
- WO-A2-2004/013160
- MANI LARIJANI ET AL: "The mutation spectrum of purified AID is similar to the mutability index in Ramos cells and in ung-/-msh2-/- mice", IMMUNOGENETICS, SPRINGER, BERLIN, DE, vol. 56, no. 11, 1 February 2005 (2005-02-01), pages 840-845, XP019331577, ISSN: 1432-1211, DOI: 10.1007/S00251-004-0748-0
- LICHTY J J ET AL: "Comparison of affinity tags for protein purification", PROTEIN EXPRESSION AND PURIFICATION, ACADEMIC PRESS, SAN DIEGO, CA, vol. 41, no. 1, 1 May 2005 (2005-05-01), pages 98-105, XP004817046, ISSN: 1046-5928, DOI: 10.1016/J.PEP.2005.01.019
- JENNY R J ET AL: "A critical review of the methods for cleavage of fusion proteins with thrombin and factor Xa", PROTEIN EXPRESSION AND PURIFICATION, ACADEMIC PRESS, SAN DIEGO, CA, vol. 31, no. 1, 1 September 2003 (2003-09-01), pages 1-11, XP004454310, ISSN: 1046-5928, DOI: 10.1016/S1046-5928(03)00168-2
- SHAHRAVAN S H ET AL: "Enhancing the specificity of the enterokinase cleavage reaction to promote efficient cleavage of a fusion tag", PROTEIN EXPRESSION AND PURIFICATION, ACADEMIC PRESS, SAN DIEGO, CA, vol. 59, no. 2, 1 June 2008 (2008-06-01), pages 314-319, XP022637192, ISSN: 1046-5928, DOI: 10.1016/J.PEP.2008.02.015 [retrieved on 2008-03-05]
- BRAR ET AL: "Activation-induced deaminase, AID, is catalytically active as a monomer on single-stranded DNA", DNA REPAIR, ELSEVIER, AMSTERDAM, NL, vol. 7, no. 1, 3 December 2007 (2007-12-03), pages 77-87, XP022373641, ISSN: 1568-7864, DOI: 10.1016/J.DNAREP.2007.08.002

## Description

The subject of invention is a composition showing AID activity, and a method of preparation of such composition in a bacterial system. In particular, the invention concerns a modified gene of human AID for the production of an active enzyme in a bacterial system as well as use of such composition for the analyses of DNA/RNA amination/deamination and/or methylation/demethylation.

Activation-induced cytidine deaminase (AID) is a key protein involved in antibody diversification process. An unusual feature of vertebrate immune system is an ability to produce large amounts of different antibodies, considerably more than the encoding genes. It is possible due to additional mechanisms that increase the level of genetic variability. Basic mechanism of the variability is somatic recombination that occurs between V (variable), D (diversity), and J (joining) genes that encode the heavy and light chains of immunoglobulins. Such recombination allows to create a primary, independent from the antigen presence set of antibodies. As a result of B-lymphocytes activation by an antigen, secondary diversification of the immunoglobulin genes occurs by way of somatic hypermutation (SHM) and class switch recombination (CSR). The SHM is a process where point mutations are generated, which induces the changes in amino acid residues that form a domain responsible for an antigen binding. CSR involves the DNA double-strand breaks and recombination that induces changes in effector (constant) region of the antibody heavy chain. As a result, the immunoglobulins that represent different classes (IgG, IgE, IgD and IgA) are formed out of the primary M type immunoglobulins (IgM) [1]. The classes have different affinity for cell receptors and, consequently, they play different roles in activating the pathways of immune response. Molecular basis for SHM and CSR had been unknown until the end of the 20^{th} century when the studies of T. Honjo's group led to the discovery of AID protein [2] and showed that it plays a key role in the initiation of both of the above- mentioned processes [3]. In the patients with mutated AID, severe immunological disorders were diagnosed: lack of somatic hypermutation, lack of class switch recombination (hyper IgM syndrome; HIGM2), and lymphoid hyperplasia [4]. The role of AID in proper immune response is crucial; however, the up-to-date data indicate that the protein may play a more important role beyond immune system. There are several lines of evidence that AID participates in genome demethylation. Presently, it is known that genome methylation is one of the critical epigenetic factors that determine gene expression. Methylation of DNA occurs mainly at 5C-position of cytosine, which leads to the formation of 5-methylcytosine (5meC). In mammals, a number of enzymes have been identified that are responsible for both de novo methylation and maintenance of methylation during DNA replication [5]. Much less is known about the molecular basis of demethylation process. It may occur passively (where the methyl groups are not incorporated into new DNA strands during replication) or in an active way (when methyl groups are removed from DNA). Active, global genome demethylation occurs in mammalian development at two distinct stages: in the early embryo immediately after fertilization and in primordial germ cells between 11.25 and 13.5 day after fertilization [6]. Demethylation restores a pluripotency state and leads to the reprogramming of the genome, thus it is essential for differentiation and development [7]. The results of the latest studies suggest that AID may be involved in this essential process [7, 8].

Although AID was discovered fifteen years ago, still little is known about its structure and catalytic mechanism. AID is a 24 kDa protein that belongs to the AID/APOBEC protein family. It has a strong nuclear export signal at the carboxyl terminus and a weak nuclear localization signal at the amino terminus. Accordingly, AID localizes mostly to the cytoplasm [9].

Similar to other proteins of the family, AID has a highly conserved amino acid motif that coordinates zinc atom inside the catalytic center. Therefore, it may be assumed that the mechanism of the cytidine to uridine deamination catalyzed by AID is similar to other zinc-dependent deaminases [10]. The closest AID homolog is the APOBEC1 protein - an enzyme that catalyzes cytidine deamination within mRNA encoding apolipoprotein B. Based on the enzymes' similarity, mRNA was also considered to be a substrate for AID [3, 9, 1 1]. The issue was intensively studied and the results showed inconsistency [12]. However, most of the data indicate that AID deaminates cytidine in a single stranded DNA [9, 13, 14], preferable within the WRCY motif (W = A/T; R = A/G; Y = C/T) [15]. AID/APOBEC is the only known human protein family capable of having direct mutagenic effect on a genome. Thus, AID overexpression leads to disorders in immune response [16] or induces tumorigenesis [17]. Some data suggest that the AID in vitro activity depends on the presence of ribonuclease, which may indicate that AID strongly binds to cellular RNA. After isolation, AID may bind to the RNA present in the preparation, which inhibits enzyme's interaction with a substrate [15]. As it was mentioned above, several issues concerning the AID structure and activity remain unknown. For instance, an ability of AID to deaminate free cytidine or deoxycytidine has been an area of controversy as some results confirm such activity [2, 15], while the others suggest the opposite [18]. Consequently, it is not possible to unequivocally establish whether the AID activity is inhibited by cytidine deaminase inhibitor - tetrahydrouridine [10]. The 3D structure of AID is unknown and all information concerning it has been based on the data referring to other enzymes of this family and bioinformatic models [10, 19]. Furthermore, it has not been established if AID occurs as a monomer [20] or - similar to Apobec2 - tetramer [19, 21], and it has not been agreed whether AID acts processively (is capable of deaminating several cytidines after binding to a substrate DNA) [22], or distributively (dissociates after a single deamination) [23]. Also, it is crucial to explain the regulation of AID activity - its expression, subcellular localization, and targeting to specific regions with simultaneous protection of other genome fragments from undesired mutations.

Unfortunately, the lack of efficient system for AID production hinders the exploration of AID structure and biochemical activity. Despite the fact that AID plays crucial roles and is an interesting target for many researchers worldwide, the enzyme remains commercially unavailable. Several papers have been published on AID production: (i) in bacterial system as a fusion protein with glutathione S-transferase (GST) [2, 24] or with streptavidin tag [18]; (ii) in insect cells [25]; and (iii) in in vitro translation system [26]. The most efficient and cheapest is bacterial system, thus it is optimal in the production of large amounts of the enzyme for structural (including crystallographic) and biochemical studies (including the testing of the compounds that modulate the enzyme activity). Based on literature, it may be assumed that the recombinant AID obtained thus far in a bacterial system was contaminated with bacterial deaminase. In fact, it enabled the study on deaminase activity of bacterial enzymes and not human AID [10]. As a result, in several publications, contrary information occurred on AID functioning, among others concerning AID ability to deaminate free nucleoside or AID activity inhibition in the presence of tetrahydrouridine.

Patent application US 2012/0151613 A1 (published 14 June 2012) presents functional mutants of human AID that have at least tenfold increased enzymatic activity than the wild type protein. The mentioned mutants were obtained in bacterial and eukaryotic systems. The patent application JP2004033137 A (published 5 February 2004) discloses the mammalian cell culture expressing human AID as a certain stage of a method to study potential anticancer compounds. Thus far, no efficient and economic method for the production of human AID that would enable its large-scale manufacture has been presented.

Furthermore, no efficient method for the production of a composition showing human AID activity in a bacterial system has been yet elaborated and patented. The aim of the invention was to obtain a modified gene of human AID that enables the production of an active AID enzyme in a bacterial system. A unique feature of the method is a modified gene of human AID. In order to obtain the gene, the following have been conducted: (i) use of the entire cDNA sequence encoding human AID except for the 5' and 3' untranslated regions, (ii) use of the sequences encoding two tags: GST and hexahistidine; (iii) use of the sequences encoding two motifs recognized by proteases: thrombin and enterokinase; (iv) assembling of the sequences encoding particular elements of the fusion protein in the following order (from the end 5' to 3'): GST tag, a motif recognized by thrombin, hexahistidine tag, a motif recognized by enterokinase, AID. Moreover, a method has been elaborated to obtain in a bacterial system a composition showing high AID activity. Such activity is defined as an ability for deamination of both cytidine and 5-methylcytidine.

Surprisingly, it appeared that the modified gene introduced in an expression vector enables efficient production of AID in a bacterial system. Moreover, the protein production process involves a unique medium composition and optimized temperature conditions for the bacteria culture. In a process that does not involve the above-mentioned factors, an enzyme is produced inefficiently or is inactive. Also, it was excluded that the observed activity derives from bacterial deaminase.

The proposed solution shall give new possibilities for thorough characterization of AID structure and biochemical activity. It will also enable the development of specific AID inhibitors that may be potentially applied for the therapy of disorders caused by increased level of AID expression, i.e. certain tumors and infections with hepatitis C virus or Eppstein-Barr virus [16, 17, 27]. Due to its high efficiency, the proposed method may be applied in commercial production of AID. The composition showing the activity of human AID (for methylated and unmethylated substrate) may find use as a potential tool for amination/deamination and methylation/demethylation research.

The invention discloses a modified gene of human activation-induced cytidine deaminase (AID), characterized in that it comprises (i) the entire cDNA sequence encoding human AID with STOP codons, except for the 5' and 3' untranslated regions, including positions 80-679 in the sequence of SEQ. ID No 1 (ii) the sequences encoding two tags: GST and hexahistidine; (iii) the sequences encoding two motifs recognized by protease: thrombin and enterokinase; (iv) the sequences encoding particular fusion protein elements in the following order (from the end 5' to 3'): GST tag, a motif recognized by thrombin, hexahistidine tag, a motif recognized by enterokinase, AID, being the sequence of SEQ. ID No 3 or the sequences showing at least 85% amino acid similarity to SEQ. ID No 4.

The invention discloses a method of modification of human AID gene characterized in that it (i) uses the entire cDNA sequence encoding human AID with STOP codons, except for the 5' and 3' untranslated regions, including positions 80-679 in a sequence SEQ. ID No 1 (ii) uses the sequences encoding two tags: GST and hexahistidine; (iii) uses the sequences encoding two motifs recognized by the proteases: thrombin and enterokinase; (iv) assemblies the sequences encoding particular elements of fusion protein in the following order (from the end 5' to 3'): GST tag, a motif recognized by thrombin, hexahistidine tag, a motif recognized by enterokinase, AID, being the sequence of SEQ. ID No 3 or the sequences showing at least 85% amino acid similarity to SEQ. ID No 4.

The invention discloses a composition showing AID activity for methylated and unmethylated cytidine, characterized in that it contains a protein produced as a result of the expression in bacterial cells of the modified gene, described above.

The subject of the invention is a method of preparation in a bacterial system of a composition showing AID activity and the method comprises an expression of a modified human AID gene in bacterial cells wherein the modified gene comprises: (i) the entire cDNA sequence encoding human AID with STOP codons, except for the 5' and 3' untranslated regions, including positions 80-679 in the sequence SEQ. ID No 1; and (ii) the sequences encoding two tags: GST and hexahistidine; and (iii) the sequences encoding two motifs recognized by proteases: thrombin and enterokinase; and (iv) the sequences encoding particular fusion protein elements in the following order (from the end 5' to 3'): GST tag, a motif recognized by thrombin, hexahistidine tag, a motif recognized by enterokinase, AID; being the sequence of SEQ. ID No 3 or the sequences showing at least 85% amino acid similarity to SEQ. ID No 4; and wherein the bacterial culture is grown on a medium that contains a source of zinc ions in the temperature conditions between 15 and 30°C, preferably 18°C.

Preferably the source of zinc ions is zinc chloride at 0.01 to 2 mM concentration, preferably 0.06 mM.
Another subject of the invention is use of the composition described above showing AID activity for methylated and/or unmethylated cytidine in amination/deamination and methylation/demethylation research.

In order to better illustrate the discussed issues, the solutions are presented in the Figure, where:

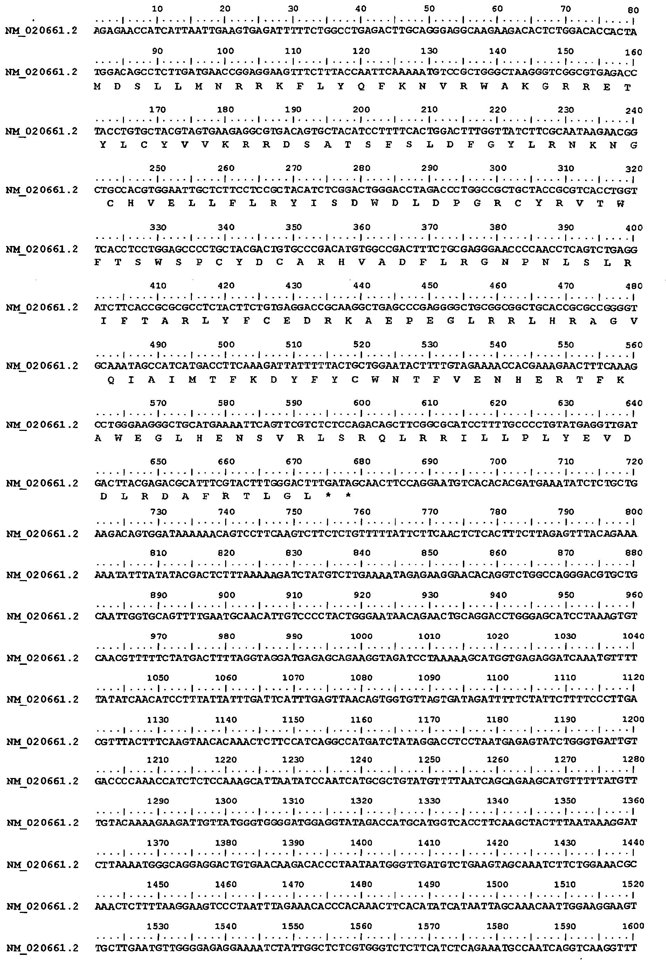

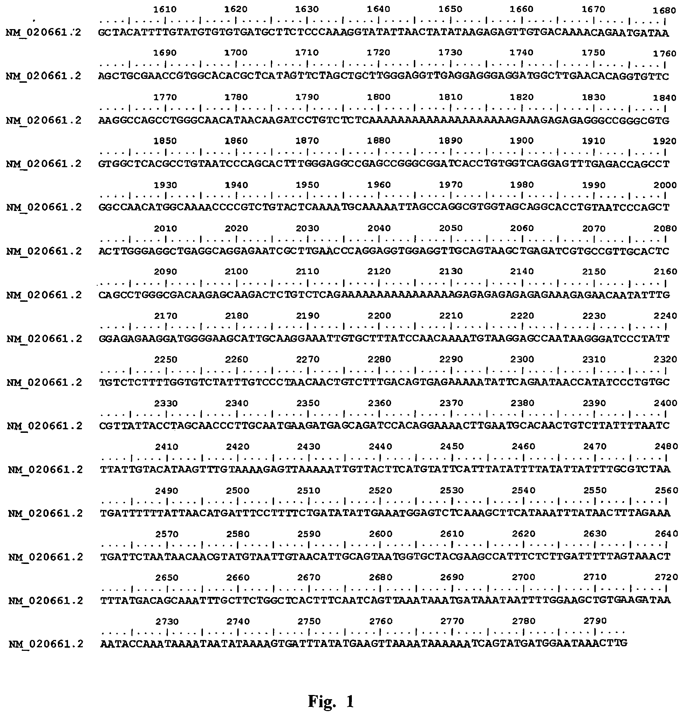

**Figure 1** and the sequence SEQ. ID No 1 presents the cDNA sequence of human AID deposited in GenBank No NM 020661.2. Within the cDNA, the coding region includes the positions 80-673 (amino acid sequence is visualized below the nucleotide sequence and shown as SEQ. ID No 2), and, then, two STOP codons occur. In the solution, the entire cDNA sequence encoding human AID was used with STOP codons (positions 80-679), except for the 5' and 3' untranslated regions;

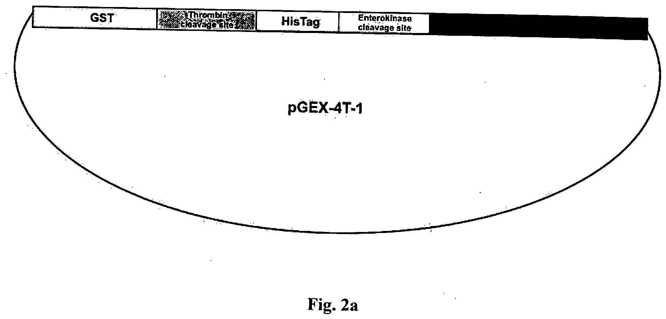

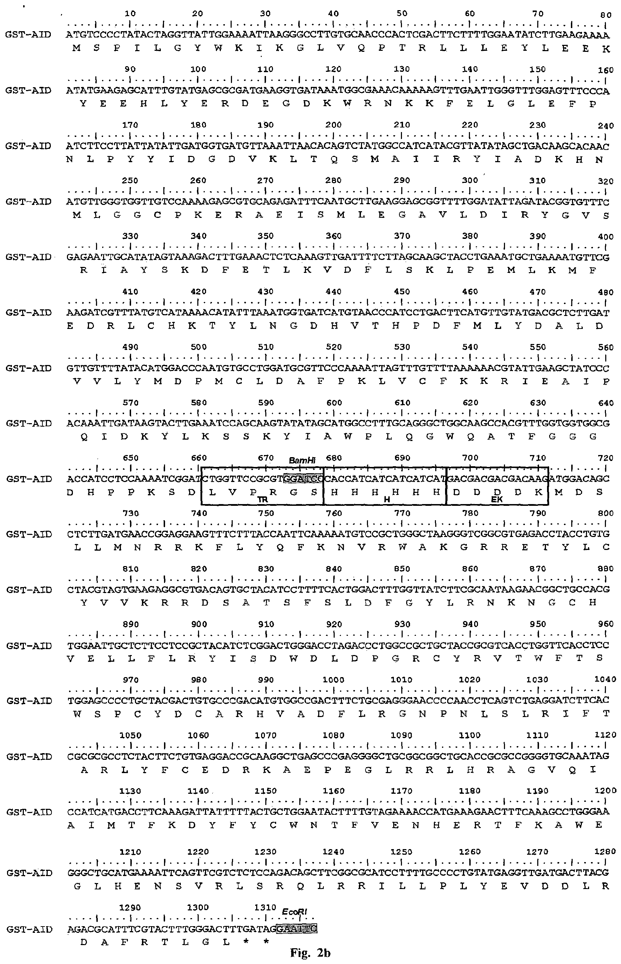

**Figure 2** presents (A) a construct scheme for the expression of human AID in a bacterial system. The construct includes the sequences encoding: (i) GST tag, (ii) a motif recognized by thrombin, (iii) hexahistidine tag, (iv) a motif recognized by enterokinase, (v) human AID; (B) nucleotide and amino acid sequence of GST-AID. The sequence encoding GST includes the positions 1-660, the sequence encoding AID includes the positions 712-1305. The sequences encoding the motif recognized by thrombin (TR), hexahistidine tag (H), and the motif recognized by enterokinase (EK) were marked with black frames. The motifs recognized by BamHI and EcoRI restriction endonucleases that were used in cloning procedure were marked with grey rectangles. The sequence was shown as SEQ ID No 3 and SEQ. ID No 4;

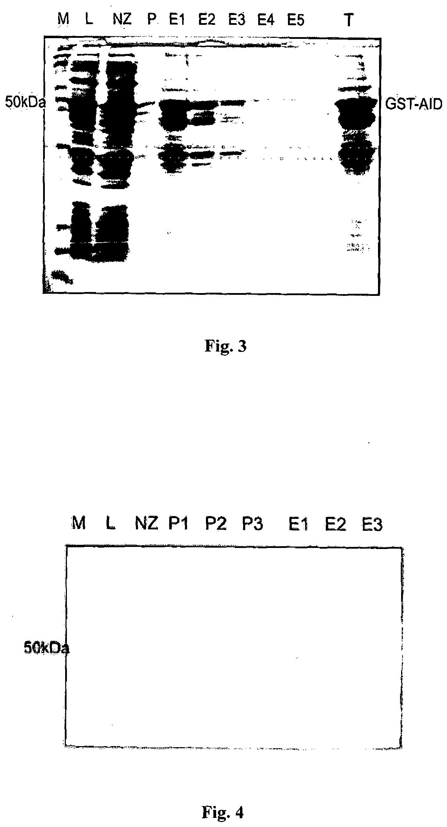

**Figure 3** presents the electrophoretic analysis in polyacrylamide gel of the fractions obtained as a result of GST-AID protein purification by glutathione affinity chromatography. M - weight marker, L - soluble proteins extract, NZ - fraction of the proteins not bound to the resin, P - fraction of the proteins eluted from the resin during washing with a glutathione-free buffer, E1-E5 - fractions of the proteins eluted from the resin during the following stages of elution with glutathione-containing buffer, T- fraction after buffer exchange to 50 mM TrisHCl including 3% glycerol. El and T fractions were used in the deaminase activity tests. The localization of GST-AID protein, with the expected mass around 50 kDa, was marked in the Figure;
**Figure 4** presents Western Blot analysis of the protein fractions obtained in the process of GST-AID purification by glutathione affinity chromatography. M - weight marker, L - soluble proteins extract, NZ - fraction of the proteins not bound to the resin, PI to P3 - fraction of the proteins eluted from the resin during washing with a glutathione-free buffer, E1-E3 - fractions of the proteins eluted from the resin with glutathione-containing buffer;

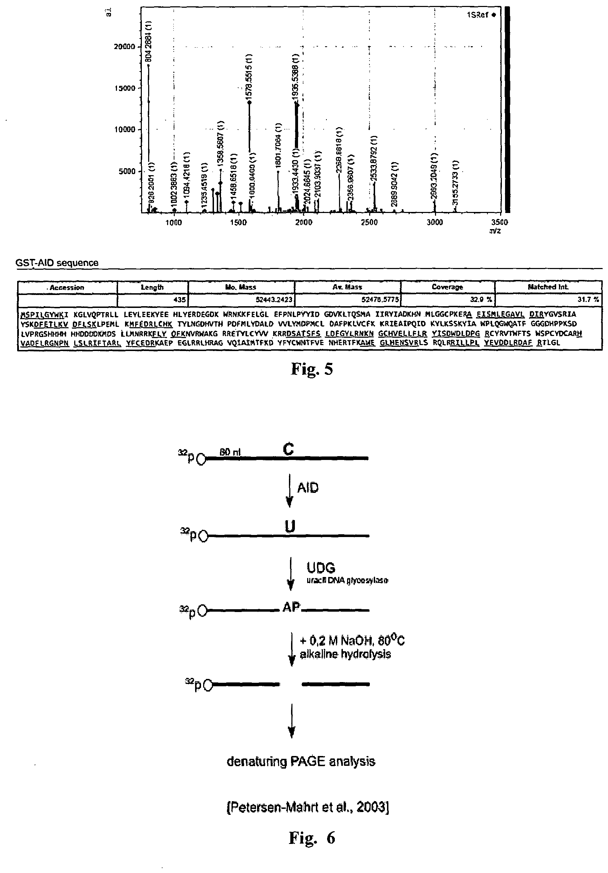

**Figure 5** presents the results of MALDI TOF analysis of the protein with the mass approx. 50 kDa eluted from a fragment of polyacrylamide gel after the analysis of the fraction obtained in the process of GST- AID elution from glutathione resin (El fraction). The discovered peptides were marked on the diagram with green dots. At the bottom, in GST- AID amino acid sequence, the discovered peptides were underlined;
**Figure 6** presents the scheme for evaluation of deaminase activity of the obtained protein composition. In the first stage, the protein composition was incubated for 30 min at 37°C with a radioactively labelled 80-nucleotide ssDNA molecule comprising cytidine at position 40. In the second stage, 2 U uracil-DNA glycosylase (UDG) was added and the reaction was incubated for additional 30 min at 37°C. In the third stage, alkaline hydrolysis was performed by 15 min incubation at 80°C in the presence of 0.2 M NaOH final concentration. The final reaction product was analyzed by electrophoresis in a denaturing polyacrylamide gel. The expected reaction product was a 40-nucleotide ssDNA;

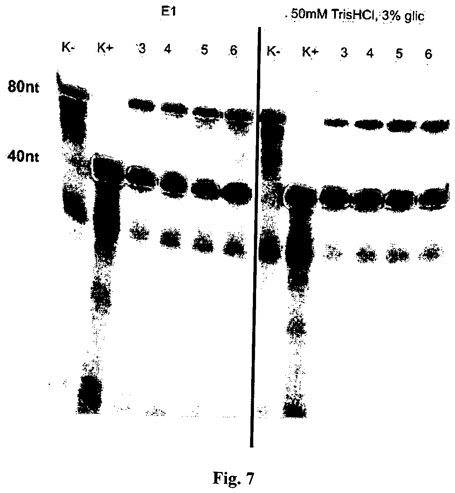

**Figure 7** presents the electrophoretic analysis of products of deamination activity assay performed for the GST- AID protein compositions in a form of fraction after the elution (El) and the fractions after buffer exchange (50 mM Tris-HCl, 3% glycerol). The content of the reaction compositions in particular lanes numbered from 3 to 6, and (K-) and (+) were presented in Table 2. (K-) negative control, (+) positive control. The 80-nucleotide substrate and 40-nucleotide reaction product are marked;

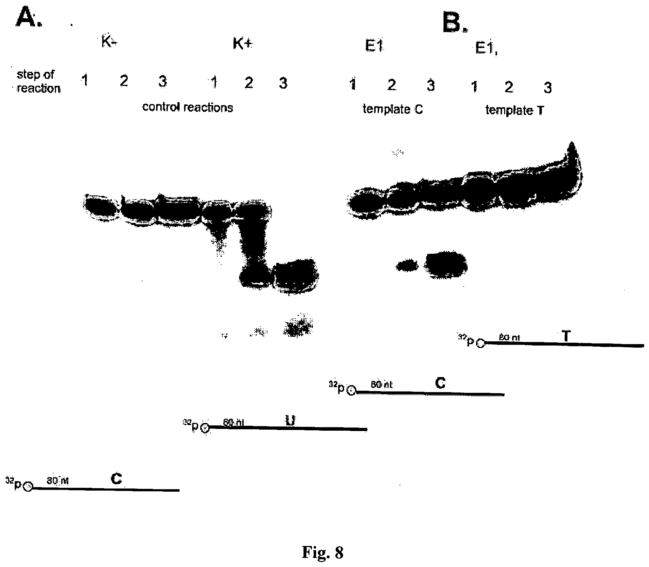

**Figure 8** presents (A) the analysis of the reaction products at each of the three stages of the activity assay (reaction stages were marked 1 to 3). The analysis was carried out for control reactions and reactions with protein composition from fraction El, and (B) the test of El fraction activity against substrate without cytidine. The analysis was carried out after each of the three stages;

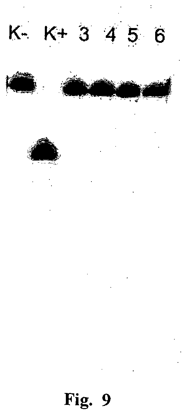

**Figure 9** presents the electrophoretic analysis of the products of deamination activity assay performed for GST-AID-N51A protein composition in a form of a fraction after the elution (El). The composition of the reaction mixtures in particular lanes numbered from 3 to 6 and of negative control (K-) and positive control (K+) was shown in Table 2. The 80- nucleotide substrate is visualized. The lack of 40-nucleotide reaction product indicates the lack of deaminase activity of the composition containing GST-AID-N51A on an unmethylated substrate.

In order to better understand the invention, an example solution is presented below.

### Example 1

In order to obtain an expression construct for the production of human AID in a bacterial system, a cDNA of human AID cloned in pOTB7 vector (Genecopoeia) was used. The clone was sequenced using BigDye Terminator vl .1 Sequencing Kit (Applied Biosystems), according to the manufacturer's protocol. The read-out was carried out using ABI-PRISM sequencer. The obtained sequence was compared with the sequences of human AID deposited in GenBank database. As a result, identity of AID sequence in pOTB7 vector (pOTB7-AID) with the sequence deposited in GenBank No NM 020661 was confirmed. It was found that it was a wild type gene, free from mutations characteristic for HIGM2 syndrome. The pOTB7-AID vector was used as a template in PCR reaction carried out in order to amplify the sequence encoding human AID.

According to literature, AID might be considered difficult in production and purification. In order to facilitate this process, a pGEX-4T-1 expression vector (GE Healthcare) was used, comprising a GST coding sequence (Fig. 2A). The connection of AID and GST shall considerably increase solubility of the produced protein and facilitate its purification. The cloning site in this plasmid is located downstream the GST encoding sequence and a short linker encoding the amino acid sequence recognized by thrombin. The introduction of an insert into the same reading frame enables the production of a fusion protein and, then, its purification by affinity chromatography to glutathione. In addition, within the insert a sequence encoding hexahistidine tag and a sequence encoding the motif recognized by enterokinase were introduced. The presented strategy allows the production of a protein with a GST tag and hexahistidine tag and, consequently, the use of two independent purification methods: affinity chromatography to glutathione and nickel ions. Moreover, the sites recognized by thrombin and enterokinase enable the cleavage of both tags and obtaining native protein. A unique feature of the proposed solution is the construction of a modified human AID gene, which involves the following: (i) use of entire cDNA sequence encoding human AID, except for the 5' and 3' untranslated regions, (ii) use of two tags: GST and hexahistidine; (iii) assembly of the sequences encoding particular elements of the fusion protein (from the end 5' to 3'): GST tag, a motif recognized by thrombin, hexahistidine tag, a motif recognized by enterokinase, AID (Fig. 2B). In order to obtain an insert encoding AID with a sequence encoding hexahistidine tag and a sequence encoding a motif recognized by enterokinase, a PCR reaction was carried out using the primers: F_03_aid, F_04_aid, R_03_aid and pOTB7-AID vector as a template. The primers' sequences were presented in Table 1. Due to considerable length of additional sequences attached to the sequence encoding AID, the PCR was carried out in two stages. In the first stage two primers were used; (i) F_03_aid primer, homologous to 5' end of the amplified gene fragment, which introduced the sequence encoding the motif recognized by enterokinase, and (ii) R_03_aid primer, complementary to 3' end of the amplified gene fragment, which introduced a site recognized by EcoRI endonuclease.

**Table 1. Sequences of the primers used**

| **Name** | **Sequence 5'-3'** |
|---|---|
| **F_03_aid** | **GACGACGACGACAAGATGGACAGCCTCTTGATGAAC** |
| **F_04_aid** | **ATGGATCCCACCATCATCATCATCATGACGACGACGACAAGATGGA** |
| **R_03_aid** | **ATGAATTCCTATCAAAGTCCCAAAGTACGA** |
| **mutAID_F** | **CTTTGGTTATCTTCGCGCTAAGAACGGCTGCC** |
| **mutAID_R** | **GAAACCAATAGAAGCGCGATTCTTGCCGACGG** |

In the second stage, the PCR product was diluted hundredfold and F_04_aid primer was used, which introduced a sequence encoding hexahistidine tag and a site recognized by BamHI endonuclease. As the second primer, R_03_aid was used once more. Both PCR reactions were carried out in 50 µl using 2 U DNA Pfu high fidelity polimerase (Promega), in the presence of 1 x PCR buffer (Promega), the 0.2 mM mixture of four deoxyribonucleoside triphosphates (dATP, dGTP, dCTP, dTTP), pOTB7-AID template in the amount of 10 ng per reaction, and the above-mentioned primers - 10 pmol per reaction. The PCR reactions were carried out using the following program: stage one - incubation at 95°C for 2 min; stage two - thirty cycles, including: incubation at 95°C for 1 min, incubation at 57°C for 30 sec, incubation at 72°C for 2 min; stage three - incubation at 72°C for 7 min, ended up with cooling of the reaction mixture to 4°C. The PCR reaction products were analyzed by agarose gel electrophoresis at native conditions, and subsequently were digested with BamHI and EcoRI restriction enzymes. The same enzymes were used for pGEX-4T-1 plasmid digestion. The substrates for ligation reaction obtained in this way were analyzed by agarose gel electrophoresis at native conditions. In the next stage, the prepared plasmids and inserts were ligated using 1 U ligase DNA T4 (Fermentas). The reaction was carried out in 10 µl in the presence of 1 x ligation buffer (Fermentas), 100 ng of vector and 50 ng of insert for 16h at 4°C. The E. coli DH5a competent cells were transformed with the ligation products. The transformed bacteria were cultivated on plates with Luria Broth medium (2 g of pepton; 1.25 g of yeast extract; 1.25 g of sodium chloride; 3.75 g of agar per 250 ml of medium), containing ampicillin at the concentration of 200 g/ml. The single colonies obtained were used for the preparation of liquid cultures on LB medium containing ampicillin, from which plasmids were isolated by alkaline lysis. The isolated plasmids were analyzed by restriction digestion with BamHI and EcoRI enzymes, followed by agarose gel electrophoresis at native conditions. As a result of plasmid digestion, 630 base pairs long dsDNA was released. The plasmids releasing the expected fragments were sequenced with BigDye Terminator vl .1 Sequencing Kit (Applied Biosystems), according to the manufacturer's protocol. The readout was carried out using ABI -PRISM sequencer. Based on this, the pGEX-4T-1-AID vector was selected (Fig. 2B) containing a proper construct encoding AID with GST tag, hexahistidine tag, and motifs recognized by proteases.

The obtained pGEX-4T-1-AID expression vector was introduced into E. coli BL21(DE3)pLysS competent cells (Novagen) by heat shock transformation. The mixture was heated for 30 sec at 42°C. The transformed cells were incubated in LB medium for 45 min at 37°C, at 250 rpm. The mixture was poured and spread on plates with LB medium containing ampicillin, and then, the plates were incubated for 16h at 37°C. A single colony was selected for preparation of liquid culture, in order to carry out AID expression. The colony was transferred into 20 ml of liquid medium containing ampicillin (200 µg/ml) and chloramphenicol (34 µg/ml), and the culture was incubated for 16h at 37°C, at 300 rpm and, next, used for inoculation of 1000 ml of fresh LB medium containing zinc chloride (0.06 mM), ampicillin (200 µg/ml), and chloramphenicol (34 µg/ml). The bacteria culture was grown at the same conditions until the absorbance OD 60o = 0.6-0.8. At that point, most intensive cell division occurs (logarithmic growth phase), thus it is an optimal moment for inducing the expression of heterologous gene. The temperature was lowered to 18°C, and AID expression was induced by adding isopropyl-P-D-1 -thiogalactopyranoside (IPTG) to the medium, to the final concentration of 0.5 mM. The expression was carried out for the following 16h. A unique feature of the proposed solution is the cultivation process: (i) in the presence of 0.06 mM zinc chloride; (ii) at 18°C. The bacterial culture was centrifuged for 15 min at 4000 rpm at 4°C. The supernatant was decanted and the pellet was suspended in PBS buffer (the content of the buffer is shown below) for the extraction of soluble proteins and their purification by glutathione affinity chromatography. For the extraction of soluble proteins, the pellet from 1 liter of the bacterial culture was suspended in 50 ml of PBS buffer (140 mM NaCl, 2.7 mM KC1, 10 mM Na 2HP0 4, 1.8 mM KH 2P0 4, pH 7.3), containing 50 U of benzonase (Novagen), protease inhibitor cocktail (Roche Diagnostics), dithiothreitol (DTT) at final concentration of 1 mM, and Triton X-100 at final concentration of 1%. The addition of protease inhibitor prevents protein degradation, and benzonase digests nucleic acids. The use of weak detergent, Triton X-100, supports protein release from cytoplasmic membranes, and reducing properties of DTT are used to hinder the formation of intermolecular disulphide bonds between cysteine residues in proteins. The suspension was incubated at 4°C for 30 min, and then, centrifuged at 12000 rpm, at 4°C for 15 min so as to separate soluble protein fractions from insoluble fractions. The GST-AID protein was found in supernatant, which was confirmed by the analysis in denaturing polyacrylamide gel. The supernatant containing the fraction of soluble proteins was then used for initial purification of the produced GST- AID protein on the glutathione resin: Glutatione Sepharose 4 Fast Flow (GE Healthcare). For protein purification, the procedure was applied according to the manufacturer's protocol. 1 ml of the resin was washed with 30 ml of PBS buffer and, next, it was incubated with protein extract for 30 min in room temperature (with gentle agitation) to facilitate protein binding to the resin. Then, it was put on chromatography column and the fraction of unbound proteins was collected. The resin was washed with 30 ml of PBS buffer in order to remove the rest of the unbound proteins. The fractions of the bound proteins were eluted by washing the column five times with 2 ml of 50 mM TrisHCl buffer, pH 8.0, containing reduced glutathione at final concentration of 10 mM. After the elution the fractions were mixed and concentrated and, next, in order to remove glutathione, the buffer was exchanged into 50 mM TrisHCl with 3% glycerol. The concentration procedure and buffer exchange were carried out using Amicon Ultra filters by MILLIPORE (15 ml; 10000 MWC), by centrifuging at 5000 rpm, at 4°C. Particular protein fractions were analyzed by electrophoresis in denaturing polyacrylamide gel (Fig. 3). The presented GST-AID purification procedure enabled the removal of considerable part of bacterial proteins. The presence of GST- AID in the purified composition was confirmed by MALDI TOF (Fig. 5.) and- Western Blot (Fig. 4.). MALDI TOF is a mass spectrometry method, widely used in proteomic studies to identify proteins. In order to carry out MALDI TOF, a fragment containing 50 kDa protein was cut out from polyacrylamide gel in which the fractions after purification on glutathione resin were analyzed. The proteins from the fragment were eluted and analyzed. The analysis confirmed that the obtained fraction contained full- length GST-AID. For the Western Blot analysis, commercially available primary rabbit polyclonal anti-AID antibody was used (Sigma, No SAB2900301). The detection was carried out with colorimetric method - secondary antibodies (goat polyclonal anti-rabbit IgG) conjugated with alkaline phosphatase were used. Both MALDI TOF and Western Blot confirmed that the produced protein is GST-AID fusion protein with expected mass of around 50 kDa. Initially purified protein composition, in the form of a fraction after elution and buffer exchange was used to characterize the enzymatic activity of GST- AID.

AID catalyzes cytidine or 5-methylcytidine deamination to, respectively, uridine or thymidine in a single-stranded DNA. In order to demonstrate that the produced protein shows the deaminase activity, a method published by Petersen-Mahrt et al. in 2002 was applied [28]. Deaminase activity of the produced protein was evaluated in a three-stage reaction (Fig. 6). As a reaction substrate, 80-nucleotide-long single-stranded DNA molecule was used, which contained only one cytidine at position 40. The molecule was radiolabelled at 5' end with γ^{[32]} P ATP in a reaction catalyzed by T4 polynucleotide kinase (Fermentas), according to the manufacturer's protocol. In the first stage, the substrate was incubated for 30 min at 37°C with the protein preparation obtained in the initial step of GST-AID elution from glutathione resin or with the protein preparation after the buffer exchange. In the first stage of the reaction, when active cytidine deaminase is present in a reaction mixture, cytidine deamination to uridine shall take place. In the second stage, 2 U uracil-DNA glycosylase was added (UDG, Fermentas) and incubated for 30 min at 37°C. The enzyme removes uracil, and apyrimidinic site is generated. In the third stage, alkaline hydrolysis was carried out by adding NaOH to the 0.2 M final concentration and incubation of the reaction mixture for 15 min at 80°C. Alkaline hydrolysis enables DNA strand-breaking at apyrimidinic site. The final products of the three-stage reaction were analyzed by electrophoresis in a denaturing polyacrylamide gel. In the presence of deaminase activity, it was expected to observe a 40-nucleotide-long product. At the same time, two control reactions were carried out: positive control reaction (+), without protein extract, but with the use of substrate molecule that had U instead of C at position 40, and negative control reaction (K-), without protein extract with the use of standard substrate that had C at position 40. While testing the GST-AID activity, four different reaction conditions were evaluated. According to literature, bacterial RNA may bind to the produced protein and inhibit its deaminase activity. Therefore, in testing the GST-AID activity, the influence of RNase on the reaction process was studied. Furthermore, it is known that AID contains a coordinated zinc ion, thus the reaction in the presence of zinc chloride was studied. Detailed composition of particular reaction mixtures is presented in Table 2. The conducted tests of GST-AID activity indicated high deaminase activity of the obtained composition. The addition of RNase or zinc did not influence the results of the reaction. The results of the product analysis carried out by electrophoresis in denaturing polyacrylamide gel are shown in Figure 7.

**Table 2. Composition of particular reaction mixtures**

| | **Trial number and composition of reaction mixture** | | | | | |
|---|---|---|---|---|---|---|
| | **(K-)** | **(K+)** | **3** | **4** | **5** | **6** |
| ZnCl₂ | 80 µm (0,8 µl) | 80 µm (0,8 µl) | - | - | 80 µm (0,8 µl) | 80 µm (0,8 µl) |
| RNase A | 1 µg (0,2 µl) | 1 µg (0,2 µl) | - | 1 µg (0,2 µl) | - | 1 µg (0,2 µl) |
| C substrate molecule | 1 pmol | - | 1 pmol | 1 pmol | 1 pmol | 1 pmol |
| U substrate molecule | - | 1 pmol | - | - | - | - |
| buffer | to 10 µl | to 10 µl | to 10 µl | to 10 µl | to 10 µl | to 10 µl |
| **AID** (fraction E1 or T) | - | - | 8 µl | 8 µl | 8 µl | 8 µl |

In order to confirm that the observed activity is deaminase activity, several additional control tests were carried out. The activity of protein preparation against the cytidine-free substrate was evaluated, the used molecule contained thymidine at position 40. The test showed lack of AID influence on cytidine-free DNA (Fig. 8). It indicates reaction specificity and shows that the observed activity is cytidine deaminase activity. Furthermore, the reaction products were tested at particular stages so as to find out at which stage the 40-nucleotide-long final product is generated. In case of contamination of the -studied preparation with nucleases, shorter DNA fragments may appear already after the first stage of the reaction. The analysis showed the presence of 80-nucleotide-long substrate after the first stage, which proves that the observed activity is not nuclease activity. The occurrence of small amounts of 40-nucleotide reaction product after the second stage may be explained by the apyrimidinic site's susceptibility to DNA strand- breaking (Fig. 8).
The obtained protein product is not homogenous. Thus, in order to exclude that the observed activity is exhibited by a bacterial enzyme, a composition that contained a mutated protein, without deaminase activity for unmethylated substrate, was prepared instead of GST- AID in the same way. In order to obtain a construct encoding the mutated AID protein, site-directed mutagenesis was applied, which enabled to introduce non- synonymous mutation into the pGEX-4T-1-AID construct obtained earlier. Previous studies showed that asparagine (N) to alanine (A) substitution at position 51 in the AID amino acid sequence leads to total loss of deaminase activity of the protein for unmethylated cytidine [26]. So as to introduce the above mutation, a PCR reaction was carried out on pGEX-4T-1-AID template, using mutAID_F and mutAID_R. primers (Table 1). Both primers were designed to introduce the mutation into a nucleotide sequence. The PCR reaction was performed to amplify the entire pGEX-4T-1-AID plasmid and to introduce, at the same time, the desired substitution in a nucleotide sequence by using the primers. The PCR reaction was conducted in two 50 µl mixtures with 2 U DNA Pfu high fidelity polymerase (Promega), in the presence of 1 x PCR buffer (Promega), the 0.2 mM mixture of four deoxyribonucleoside triphosphates (dATP, dGTP, dCTP, dTTP), 500 ng of pGEX-4T-1-AID template per reaction, and 50 pmol the above-mentioned primers per reaction. The first ten reaction cycles were carried out separately for each of the primers (each mixture contained only one primer of the pair). After the tenth cycle, both reactions were mixed, divided into two samples once more, and the amplification was continued for the next twenty cycles. The PCR reactions were carried out according to the following program: stage one - incubation at 95°C for 2 min; stage two - ten or twenty cycles, including: incubation at 95°C for 1 min, incubation at 60°C for 30 sec, incubation at 72°C for 11 min, respectively; stage three - incubation at 72°C for 15 min, and final cooling of the reaction mixture to 4°C. The PCR reaction products were analyzed by agarose gel electrophoresis at native conditions, and were then further digested by Dpnl restriction enzyme. The enzyme enables selective digestion of the template used for the PCR reaction. The product of restriction digestion was used for the transformation of E. coli TOP 10 competent cells (Invitrogen). The transformed bacteria were grown on plates with LB medium that contained ampicillin at the concentration of 200 µg ml. The obtained single colonies were used for the preparation of liquid cultures in LB medium with ampicillin, from which plasmids were isolated by alkaline lysis. The isolated plasmids were analyzed by sequencing using the BigDye Terminator vl . 1 Sequencing Kit (Applied Biosystems), according to the manufacturer's protocol. The sequence read-out was carried out with ABI- PRISM sequencer. On such basis, the plasmid was selected that contained the introduced mutation. The obtained pGEX-4T-1-AID-N51A expression vector was introduced into E. coli BL21(DE3) pLysS competent cells (Novagen) by heat shock transformation. Further stages of the protein extracts preparation were analogous to those carried out for wild type GST-AID.
GST-AID-N51A protein composition showed no deaminase activity for unmethylated substrate (Fig. 9). It was thus excluded that the previously observed activity of the preparation that contained wild type GST-AID was derived from bacterial deaminase. Moreover it was stated that both AID and mutants of AID have the ability of 5- methylcytidine deamination.

The proposed solution, including (i) a unique modification of human AID gene to produce (in bacterial system) a composition showing human AID activity and (ii) method of preparation (in bacterial system) of the composition showing AID activity, may have several practical applications. It enables to obtain large amounts of the composition showing human AID activity at considerably low expense. Therefore, it is a very advantageous solution in large-scale production of AID for commercial use (at present, the composition of that type is not commercially available). Considering the role of AID, the composition will be widely accepted by the scientists worldwide. Finally, its availability will facilitate better understanding of biochemical properties and 3D structure of AID as well as design and delivery of the molecules modulating its activity (inhibiting and stimulating), including pharmacologically active compounds. Composition having the human AID activity may also be used as the tool for DNA/RNA amination/deamination and/or methylation/demethylation research.

### References

1. Delker, R.K., S.D. Fugmann, and F.N. Papavasiliou, A coming-of-age story: activation-induced cytidine deaminase turns 10. Nat Immunol, 2009. 10(11): p. 1147-53.
2. Muramatsu, M., et al., Specific expression of activation-induced cytidine deaminase (AID), a novel member of the RNA-editing deaminase family in germinal center B cells. J Biol Chem, 1999. 274(26): p. 18470-6.
3. Muramatsu, M., et al., Class switch recombination and hypermutation require activation-induced cytidine deaminase (AID), a potential RNA editing enzyme. Cell, 2000. 102(5): p. 553-63.
4. Revy, P., et al., Activation-induced cytidine deaminase (AID) deficiency causes the autosomal recessive form of the Hyper-IgM syndrome (HIGM2). Cell, 2000. 102(5): p. 565-75.
5. Bestor, T.H., The DNA methyltransferases of mammals. Hum Mol Genet, 2000. 9(16): p. 2395-402.
6. Gehring, M., W. Reik, and S. Henikoff, DNA demethylation by DNA repair. Trends Genet, 2009. 25(2): p. 82-90.
7. Teperek-Tkacz, M., et al., Epigenetic reprogramming: is deamination key to active DNA demethylation? Reproduction, 2011. 142(5): p. 621-32.
8. Fritz, E.L. and F.N. Papavasiliou, Cytidine deaminases: AIDing DNA demethylation? Genes Dev, 2010. 24(19): p. 2107-14.
9. Conticello, S.G., The AID/APOBEC family of nucleic acid mutators. Genome Biol, 2008. 9(6): p. 229.
10. Samaranayake, M., et al., Evaluation of molecular models for the affinity maturation of antibodies: roles of cytosine deamination by AID and DNA repair. Chem Rev, 2006. 106(2): p. 700-19.
11. Honjo, T., K. Kinoshita, and M. Muramatsu, Molecular mechanism of class switch recombination: linkage with somatic hypermutation. Annu Rev Immunol, 2002. 20: p. 165-96.
12. Honjo, T., et al., AID to overcome the limitations of genomic information. Nat Immunol, 2005. 6(7): p. 655-61.
13. Barreto, V.M., A.R. Ramiro, and M.C. Nussenzweig, Activation-induced deaminase: controversies and open questions. Trends Immunol, 2005. 26(2): p. 90-6.
14. de Yebenes, V.G. and A.R. Ramiro, Activation-induced deaminase: light and dark sides. Trends Mol Med, 2006. 12(9): p. 432-9.
15. Bransteitter, R., et al., Activation-induced cytidine deaminase deaminates deoxycytidine on single-stranded DNA but requires the action of RNase. Proc Natl Acad Sci USA, 2003. 100(7): p. 4102-7.
16. Machida, K., et al., Hepatitis C virus (HCV)-induced immunoglobulin hypermutation reduces the affinity and neutralizing activities of antibodies against HCV envelope protein. J Virol, 2008. 82(13): p. 6711-20.
17. Marusawa, H., Aberrant AID expression and human cancer development. Int J Biochem Cell Biol, 2008. 40(8): p. 1399-402.
18. Dickerson, S.K., et al., AID mediates hypermutation by deaminating single stranded DNA. J Exp Med, 2003. 197(10): p. 1291-6.
19. Bhagwat, A.S., M.A. Carpenter, and J.M. Bujnicki, Is AID a monomer in solution? DNA Repair (Amst), 2008. 7(3): p. 349-50; author reply 351-2.
20. Brar, S.S., et al., Activation-induced deaminase, AID, is catalytically active as a monomer on single-stranded DNA. DNA Repair (Amst), 2008. 7(1): p. 77-87.
21. Prochnow, C., et al., The APOBEC-2 crystal structure and functional implications for the deaminase AID. Nature, 2007. 445(7126): p. 447-51.
22. Chelico, L., P. Pham, and M.F. Goodman, Stochastic properties of processive cytidine DNA deaminases AID and APOBEC3G. Philos Trans R Soc Lond B Biol Sci, 2009. 364(1517): p. 583-93.
23. Coker, H.A. and S.K. Petersen-Mahrt, The nuclear DNA deaminase AID functions distributively whereas cytoplasmic APOBEC3G has a processive mode of action. DNA Repair (Amst), 2007. 6(2): p. 235-43.
24. Papavasiliou, F.N. and D.G. Schatz, The activation-induced deaminase functions in a postcleavage step of the somatic hypermutation process. J Exp Med, 2002. 195(9): p. 1193-8.
25. Bransteitter, R., et al., Biochemical analysis of hypermutational targeting by wild type and mutant activation-induced cytidine deaminase. J Biol Chem, 2004. 279(49): p. 51612-21.
26. Shivarov, V., R. Shinkura, and T. Honjo, Dissociation of in vitro DNA deamination activity and physiological functions of AID mutants. Proc Natl Acad Sci U S A, 2008.105(41): p. 15866-71.
27. Epeldegui, M., et al., Infection of human B cells with Epstein-Barr virus results in the expression of somatic hypermutation-inducing molecules and in the accrual of oncogene mutations. Mol Immunol, 2007. 44(5): p. 934-42.
28. Petersen-Mahrt, S.K., R.S. Harris, and M.S. Neuberger, AID mutates E. coli suggesting a DNA deamination mechanism for antibody diversification. Nature, 2002. 418(6893): p. 99-103.

### SEQUENCE LISTING

<110> INSTYTUT CHEMII BIOORGANICZNEJ PAN
<120> Modified gene of human activation-induced cytidine deaminase (AID), a
   method of modification of human AID gene, a composition showing AID activity, a method of preparation of such composition in a bacterial system and use of the composition in the analyses of DNA/RNA amination/deamination and/or methylation/demethylation
<130> 18647/13
<150> PL402440
   <151> 2013-01-14
<160> 9
<170> BiSSAP 1.0
<210> 1
   <211> 2794
   <212> DNA
   <213> Homo sapiens
<220>
   <221> source
   <222> 1..2794
   <223> /mol_type="DNA" /note="NM_020661.2_nt" /organism="Homo sapiens"
<400> 1
   agagaaccat cattaattga agtgagattt ttctggcctg agacttgcag ggaggcaaga      60
   agacactctg gacaccacta tggacagcct cttgatgaac cggaggaagt ttctttacca     120
   attcaaaaat gtccgctggg ctaagggtcg gcgtgagacc tacctgtgct acgtagtgaa     180
   gaggcgtgac agtgctacat ccttttcact ggactttggt tatcttcgca ataagaacgg     240
   ctgccacgtg gaattgctct tcctccgcta catctcggac tgggacctag accctggccg     300
   ctgctaccgc gtcacctggt tcacctcctg gagcccctgc tacgactgtg cccgacatgt     360
   ggccgacttt ctgcgaggga accccaacct cagtctgagg atcttcaccg cgcgcctcta     420
   cttctgtgag gaccgcaagg ctgagcccga ggggctgcgg cggctgcacc gcgccggggt     480
   gcaaatagcc atcatgacct tcaaagatta tttttactgc tggaatactt ttgtagaaaa     540
   ccacgaaaga actttcaaag cctgggaagg gctgcatgaa aattcagttc gtctctccag     600
   acagcttcgg cgcatccttt tgcccctgta tgaggttgat gacttacgag acgcatttcg     660
   tactttggga ctttgatagc aacttccagg aatgtcacac acgatgaaat atctctgctg     720
   aagacagtgg ataaaaaaca gtccttcaag tcttctctgt tttattcttc caactctcac     780
   tttcttagag tttacagaaa aaatatttat atacgactct ttaaaaagat ctatgtcttg     840
   aaaatagaga aggaacacag gtctggccag ggacgtgctg caattggtgc agttttgaat     900
   gcaacattgt cccctactgg gaataacaga actgcaggac ctgggagcat cctaaagtgt     960
   caacgttttt ctatgacttt taggtaggat gagagcagaa ggtagatcct aaaaagcatg    1020
   gtgagaggat caaatgtttt tatatcaaca tcctttatta tttgattcat ttgagttaac    1080
   agtggtgtta gtgatagatt tttctattct tttcccttga cgtttacttt caagtaacac    1140
   aaactcttcc atcaggccat gatctatagg acctcctaat gagagtatct gggtgattgt    1200
   gaccccaaac catctctcca aagcattaat atccaatcat gcgctgtatg ttttaatcag    1260
   cagaagcatg tttttatgtt tgtacaaaag aagattgtta tgggtgggga tggaggtata    1320
   gaccatgcat ggtcaccttc aagctacttt aataaaggat cttaaaatgg gcaggaggac    1380
   tgtgaacaag acaccctaat aatgggttga tgtctgaagt agcaaatctt ctggaaacgc    1440
   aaactctttt aaggaagtcc ctaatttaga aacacccaca aacttcacat atcataatta    1500
   gcaaacaatt ggaaggaagt tgcttgaatg ttggggagag gaaaatctat tggctctcgt    1560
   gggtctcttc atctcagaaa tgccaatcag gtcaaggttt gctacatttt gtatgtgtgt    1620
   gatgcttctc ccaaaggtat attaactata taagagagtt gtgacaaaac agaatgataa    1680
   agctgcgaac cgtggcacac gctcatagtt ctagctgctt gggaggttga ggagggagga    1740
   tggcttgaac acaggtgttc aaggccagcc tgggcaacat aacaagatcc tgtctctcaa    1800
   aaaaaaaaaa aaaaaaaaga aagagagagg gccgggcgtg gtggctcacg cctgtaatcc    1860
   cagcactttg ggaggccgag ccgggcggat cacctgtggt caggagtttg agaccagcct    1920
   ggccaacatg gcaaaacccc gtctgtactc aaaatgcaaa aattagccag gcgtggtagc    1980
   aggcacctgt aatcccagct acttgggagg ctgaggcagg agaatcgctt gaacccagga    2040
   ggtggaggtt gcagtaagct gagatcgtgc cgttgcactc cagcctgggc gacaagagca    2100
   agactctgtc tcagaaaaaa aaaaaaaaaa gagagagaga gagaaagaga acaatatttg    2160
   ggagagaagg atggggaagc attgcaagga aattgtgctt tatccaacaa aatgtaagga    2220
   gccaataagg gatccctatt tgtctctttt ggtgtctatt tgtccctaac aactgtcttt    2280
   gacagtgaga aaaatattca gaataaccat atccctgtgc cgttattacc tagcaaccct    2340
   tgcaatgaag atgagcagat ccacaggaaa acttgaatgc acaactgtct tattttaatc    2400
   ttattgtaca taagtttgta aaagagttaa aaattgttac ttcatgtatt catttatatt    2460
   ttatattatt ttgcgtctaa tgatttttta ttaacatgat ttccttttct gatatattga    2520
   aatggagtct caaagcttca taaatttata actttagaaa tgattctaat aacaacgtat    2580
   gtaattgtaa cattgcagta atggtgctac gaagccattt ctcttgattt ttagtaaact    2640
   tttatgacag caaatttgct tctggctcac tttcaatcag ttaaataaat gataaataat    2700
   tttggaagct gtgaagataa aataccaaat aaaataatat aaaagtgatt tatatgaagt    2760
   taaaataaaa aatcagtatg atggaataaa cttg                                2794
<210> 2
   <211> 198
   <212> PRT
   <213> Homo sapiens
<220>
   <221> SOURCE
   <222> 1..198
   <223> /mol_type="protein" /note="NM_020661.2_aa" /organism="Homo sapiens"
<400> 2
<210> 3
   <211> 1317
   <212> DNA
   <213> Homo sapiens
<220>
   <221> source
   <222> 1..1317
   <223> /mol_type="DNA" /note="GST-AID_nt" /organism="Homo sapiens"
<400> 3
   atgtccccta tactaggtta ttggaaaatt aagggccttg tgcaacccac tcgacttctt      60
   ttggaatatc ttgaagaaaa atatgaagag catttgtatg agcgcgatga aggtgataaa     120
   tggcgaaaca aaaagtttga attgggtttg gagtttccca atcttcctta ttatattgat     180
   ggtgatgtta aattaacaca gtctatggcc atcatacgtt atatagctga caagcacaac     240
   atgttgggtg gttgtccaaa agagcgtgca gagatttcaa tgcttgaagg agcggttttg     300
   gatattagat acggtgtttc gagaattgca tatagtaaag actttgaaac tctcaaagtt     360
   gattttctta gcaagctacc tgaaatgctg aaaatgttcg aagatcgttt atgtcataaa     420
   acatatttaa atggtgatca tgtaacccat cctgacttca tgttgtatga cgctcttgat     480
   gttgttttat acatggaccc aatgtgcctg gatgcgttcc caaaattagt ttgttttaaa     540
   aaacgtattg aagctatccc acaaattgat aagtacttga aatccagcaa gtatatagca     600
   tggcctttgc agggctggca agccacgttt ggtggtggcg accatcctcc aaaatcggat     660
   ctggttccgc gtggatccca ccatcatcat catcatgacg acgacgacaa gatggacagc     720
   ctcttgatga accggaggaa gtttctttac caattcaaaa atgtccgctg ggctaagggt     780
   cggcgtgaga cctacctgtg ctacgtagtg aagaggcgtg acagtgctac atccttttca     840
   ctggactttg gttatcttcg caataagaac ggctgccacg tggaattgct cttcctccgc     900
   tacatctcgg actgggacct agaccctggc cgctgctacc gcgtcacctg gttcacctcc     960
   tggagcccct gctacgactg tgcccgacat gtggccgact ttctgcgagg gaaccccaac    1020
   ctcagtctga ggatcttcac cgcgcgcctc tacttctgtg aggaccgcaa ggctgagccc    1080
   gaggggctgc ggcggctgca ccgcgccggg gtgcaaatag ccatcatgac cttcaaagat    1140
   tatttttact gctggaatac ttttgtagaa aaccatgaaa gaactttcaa agcctgggaa    1200
   gggctgcatg aaaattcagt tcgtctctcc agacagcttc ggcgcatcct tttgcccctg    1260
   tatgaggttg atgacttacg agacgcattt cgtactttgg gactttgata ggaattc       1317
<210> 4
   <211> 435
   <212> PRT
   <213> Homo sapiens
<220>
   <221> SOURCE
   <222> 1..435
   <223> /mol_type="protein" /note="GST-AID_aa" /organism="Homo sapiens"
<400> 4
   Met Ser Pro Ile Leu Gly Tyr Trp Lys Ile Lys Gly Leu Val Gln Pro
   1               5                   10                  15
   Thr Arg Leu Leu Leu Glu Tyr Leu Glu Glu Lys Tyr Glu Glu His Leu
               20                  25                  30
   Tyr Glu Arg Asp Glu Gly Asp Lys Trp Arg Asn Lys Lys Phe Glu Leu
           35                  40                  45
   Gly Leu Glu Phe Pro Asn Leu Pro Tyr Tyr Ile Asp Gly Asp Val Lys
       50                  55                  60
   Leu Thr Gln Ser Met Ala Ile Ile Arg Tyr Ile Ala Asp Lys His Asn
   65                  70                  75                  80
   Met Leu Gly Gly Cys Pro Lys Glu Arg Ala Glu Ile Ser Met Leu Glu
                   85                  90                  95
   Gly Ala Val Leu Asp Ile Arg Tyr Gly Val Ser Arg Ile Ala Tyr Ser
               100                 105                 110
   Lys Asp Phe Glu Thr Leu Lys Val Asp Phe Leu Ser Lys Leu Pro Glu
           115                 120                 125
   Met Leu Lys Met Phe Glu Asp Arg Leu Cys His Lys Thr Tyr Leu Asn
       130                 135                 140
   Gly Asp His Val Thr His Pro Asp Phe Met Leu Tyr Asp Ala Leu Asp
   145                 150                 155                 160
   Val Val Leu Tyr Met Asp Pro Met Cys Leu Asp Ala Phe Pro Lys Leu
                   165                 170                 175
   Val Cys Phe Lys Lys Arg Ile Glu Ala Ile Pro Gln Ile Asp Lys Tyr
               180                 185                 190
   Leu Lys Ser Ser Lys Tyr Ile Ala Trp Pro Leu Gln Gly Trp Gln Ala
           195                 200                 205
   Thr Phe Gly Gly Gly Asp His Pro Pro Lys Ser Asp Leu Val Pro Arg
       210                 215                 220
   Gly Ser His His His His His His Asp Asp Asp Asp Lys Met Asp Ser
   225                 230                 235                 240
   Leu Leu Met Asn Arg Arg Lys Phe Leu Tyr Gln Phe Lys Asn Val Arg
                   245                 250                 255
   Trp Ala Lys Gly Arg Arg Glu Thr Tyr Leu Cys Tyr Val Val Lys Arg
               260                 265                 270
   Arg Asp Ser Ala Thr Ser Phe Ser Leu Asp Phe Gly Tyr Leu Arg Asn
           275                 280                 285
   Lys Asn Gly Cys His Val Glu Leu Leu Phe Leu Arg Tyr Ile Ser Asp
       290                 295                 300
   Trp Asp Leu Asp Pro Gly Arg Cys Tyr Arg Val Thr Trp Phe Thr Ser
   305                 310                 315                 320
   Trp Ser Pro Cys Tyr Asp Cys Ala Arg His Val Ala Asp Phe Leu Arg
                   325                 330                 335
   Gly Asn Pro Asn Leu Ser Leu Arg Ile Phe Thr Ala Arg Leu Tyr Phe
               340                 345                 350
   Cys Glu Asp Arg Lys Ala Glu Pro Glu Gly Leu Arg Arg Leu His Arg
           355                 360                 365
   Ala Gly Val Gln Ile Ala Ile Met Thr Phe Lys Asp Tyr Phe Tyr Cys
       370                 375                 380
   Trp Asn Thr Phe Val Glu Asn His Glu Arg Thr Phe Lys Ala Trp Glu
   385                 390                 395                 400
   Gly Leu His Glu Asn Ser Val Arg Leu Ser Arg Gln Leu Arg Arg Ile
                   405                 410                 415
   Leu Leu Pro Leu Tyr Glu Val Asp Asp Leu Arg Asp Ala Phe Arg Thr
               420                 425                 430
   Leu Gly Leu
           435
<210> 5
   <211> 36
   <212> DNA
   <213> artificial sequences
<220>
   <221> source
   <222> 1..36
   <223> /mol_type="DNA" /note="F_03_aid" /organism="artificial sequences"
<400> 5
   gacgacgacg acaagatgga cagcctcttg atgaac 36
<210> 6
   <211> 46
   <212> DNA
   <213> artificial sequences
<220>
   <221> source
   <222> 1..46
   <223> /mol_type="DNA" /note="F_04_aid" /organism="artificial sequences"
<400> 6
   atggatccca ccatcatcat catcatgacg acgacgacaa gatgga 46
<210> 7
   <211> 30
   <212> DNA
   <213> artificial sequences
<220>
   <221> source
   <222> 1..30
   <223> /mol_type="DNA" /note="R_03_aid" /organism="artificial sequences"
<400> 7
   atgaattcct atcaaagtcc caaagtacga 30
<210> 8
   <211> 32
   <212> DNA
   <213> artificial sequences
<220>
   <221> source
   <222> 1..32
   <223> /mol_type="DNA" /note="mutAID_F" /organism="artificial sequences"
<400> 8
   ctttggttat cttcgcgcta agaacggctg cc 32
<210> 9
   <211> 32
   <212> DNA
   <213> artificial sequences
<220>
   <221> source
   <222> 1..32
   <223> /mol_type="DNA" /note="mutAID_R" /organism="artificial sequences"
<400> 9
   gaaaccaata gaagcgcgat tcttgccgac gg 32

## Claims

1. A composition showing activity of human activation-induced cytidine deaminase (AID) for methylated and/or unmethylated cytidine **characterized in that** it contains a protein produced as a result of the expression of the modified gene of human AID in bacterial cells, and the modified gene comprises: (i) the entire cDNA sequence encoding human AID with STOP codons, except for the 5' and 3' untranslated regions, including positions 80-679 in a sequence SEQ. ID No 1; and (ii) sequences encoding two tags: GST and hexahistidine; and (iii) sequences encoding two motifs recognized by the proteases: thrombin and enterokinase; and (iv) sequences encoding particular elements of fusion protein in the following order (from the end 5' to 3'): GST tag, a motif recognized by thrombin, hexahistidine tag, a motif recognized by enterokinase, AID; all being the sequence SEQ. ID No 3 or the sequences showing at least 85% amino acid similarity to SEQ. ID No 4;
wherein the bacterial cell culture is grown in a medium that contains a source of zinc ions and the bacterial cell culture is grown at the temperature from 15 to 30°C, preferably 18 °C.

2. A method of preparation in a bacterial system of a composition showing activity of human activation-induced cytidine deaminase (AID) for methylated and/or unmethylated cytidine, and the method comprises an expression of a modified gene of human AID in bacterial cells,
wherein the modified gene comprises: (i) the entire cDNA sequence encoding human AID with STOP codons, except for the 5' and 3' untranslated regions, including positions 80-679 in a sequence SEQ. ID No 1, and (ii) the sequences encoding two tags: GST and hexahistidine; and (iii) the sequences encoding two motifs recognized by the proteases:
thrombin and enterokinase; and (iv) the sequences encoding particular elements of fusion protein in the following order (from the end 5' to 3'): GST tag, a motif recognized by thrombin, hexahistidine tag, a motif recognized by enterokinase, AID; all being the sequence SEQ. ID No 3 or the sequences showing at least 85% amino acid similarity to SEQ. ID No 4;
and wherein the bacterial cell culture is grown in a medium that contains a source of zinc ions and the bacterial cell culture is grown at the temperature from 15 to 30°C, preferably 18°C.

3. The method according to claim 2, **characterized in that** the source of zinc ions is zinc chloride at the concentration from 0.01 to 2 mM, preferably 0.06 mM.

4. Use of the composition as defined in claim 1 in the analyses of DNA/RNA amination/deamination and methylation/demethylation.

## Patentansprüche

1. Eine Zusammensetzung mit Aktivität menschlicher aktivierungsinduzierter Cytidindeaminase (AID) für methyliertes und/oder unmethyliertes Cytidin, dadurch charakterisiert, dass sie ein Protein enthält, das als Ergebnis der Expression des modifizierten Gens menschlicher AID in bakteriellen Zellen hergestellt wird, und dass das modifizierte Gen umfasst: (i) die gesamte cDNA-Sequenz, die menschliche AID mit STOP-Codons kodiert, ausser den 5'- und 3'-nichttranslatierten Regionen, einschließlich Positionen 80-679 von Sequenz SEQ ID NO:1; und (ii) Sequenzen, die zwei Tags kodieren: GST und Hexahistidin; und (iii) Sequenzen, die zwei von den Proteasen Thrombin und Enterokinase erkannte Motive kodieren; und (iv) Sequenzen, die bestimmte Elemente von Fusionsproteinen in folgender Reihenfolge kodieren (vom 5'-zum 3'-Ende): GST-Tag, ein von Thrombin erkanntes Motiv, Hexahistidin-Tag, ein von Enterokinase erkanntes Motiv, AID; das alles sind die Sequenz SEQ ID NO:3 oder die Sequenzen mit wenigstens 85% Aminosäureähnlichkeit mit SEQ ID NO:4;
wobei die bakterielle Zellkultur bei einer Temperatur von 15 bis 30°C, vorzugsweise 18°C, in einem Medium gezüchtet wird, das eine Zinkionenquelle enthält.

2. Ein Verfahren zur Herstellung einer Zusammensetzung mit Aktivität menschlicher aktivierungsinduzierter Cytidindeaminase (AID) für methyliertes und/oder unmethyliertes Cytidin in einem bakteriellen System, wobei das Verfahren eine Expression eines modifizierten Gens menschlicher AID in bakteriellen Zellen umfasst,
wobei das modifizierte Gen umfasst: (i) die gesamte cDNA-Sequenz, die menschliche AID mit STOP-Codons kodiert, ausser den 5'- und 3'-nichttranslatierten Regionen, einschließlich Positionen 80-679 von SEQ ID NO:1; und (ii) Sequenzen, die zwei Tags kodieren: GST und Hexahistidin; und (iii) Sequenzen, die zwei von den Proteasen Thrombin und Enterokinase erkannte Motive kodieren; und (iv) Sequenzen, die bestimmte Elemente von Fusionsproteinen in folgender Reihenfolge kodieren (vom 5'-zum 3'-Ende): GST-Tag, ein von Thrombin erkanntes Motiv, Hexahistidin-Tag, ein von Enterokinase erkanntes Motiv, AID; das alles sind die Sequenz SEQ ID NO:3 oder die Sequenzen mit wenigstens 85% Aminosäureähnlichkeit mit SEQ ID NO:4;
und wobei die bakterielle Zellkultur bei einer Temperatur von 15 bis 30°C, vorzugsweise 18°C, in einem Medium gezüchtet wird, das eine Zinkionenquelle enthält.

3. Das Verfahren gemäß Anspruch 2, dadurch charakterisiert, dass die Zinkionenquelle Zinkchlorid bei einer Konzentration von 0.01 bis 2 mM ist, vorzugsweise 0.06 mM.

4. Verwendung der Zusammensetzung wie in Anspruch 1 definiert in der Analyse von DNA/RNA-Aminierung/-Deaminierung und -Methylierung/-Demethylierung.

## Revendications

1. Composition présentant une activité de cytidine-désaminase induite par activation (AID, *« Activation*-*induced cytidine deaminase* ») humaine pour de la cytidine méthylée et/ou non méthylée **caractérisée en ce qu'**elle contient une protéine produite par suite de l'expression du gène modifié de l'AID humaine dans des cellules bactériennes, et le gène modifié comprenant : (i) la totalité de la séquence d'ADNc codant pour l'AID humaine avec des codons STOP, à l'exception des régions non traduites en 5' et 3', y compris les positions 80-679 dans une séquence SEQ. ID N° 1 ; et (ii) des séquences codant pour deux marqueurs : GST et hexahistidine ; et (iii) des séquences codant pour deux motifs reconnus par les protéases : thrombine et entérokinase ; et (iv) des séquences codant pour des éléments particuliers de la protéine de fusion dans l'ordre suivant (de l'extrémité 5' à 3') : marqueur GST, un motif reconnu par la thrombine, marqueur hexahistidine, un motif reconnu par entérokinase, AID ; le tout étant la séquence SEQ. ID N° 3 ou les séquences présentant une similitude d'acides aminés d'au moins 85 % avec la SEQ. ID N° 4 ; dans laquelle la culture de cellules bactériennes est cultivée dans un milieu qui contient une source d'ions zinc et la culture de cellules bactériennes est cultivée à la température de 15 à 30 °C, de préférence 18 °C.

2. Procédé de préparation dans un système bactérien d'une composition présentant une activité de cytidine-désaminase induite par activation (AID, *« Activation*-*induced cytidine deaminase* ») humaine pour de la cytidine méthylée et/ou non méthylée, et le procédé comprenant une expression d'un gène modifié de l'AID humaine dans des cellules bactériennes,
dans lequel le gène modifié comprend : (i) la totalité de la séquence d'ADNc codant pour l'AID humaine avec des codons STOP, à l'exception des régions non traduites en 5' et 3', y compris les positions 80-679 dans une séquence SEQ. ID N° 1 ; et (ii) les séquences codant pour deux marqueurs : GST et hexahistidine ; et (iii) les séquences codant pour deux motifs reconnus par les protéases : thrombine et entérokinase ; et (iv) les séquences codant pour des éléments particuliers de la protéine de fusion dans l'ordre suivant (de l'extrémité 5' à 3') : marqueur GST, un motif reconnu par la thrombine, marqueur hexahistidine, un motif reconnu par l'entérokinase, AID ; le tout étant la séquence SEQ. ID N° 3 ou les séquences présentant une similitude d'acides aminés d'au moins 85 % avec la SEQ. ID N° 4 ;
et dans lequel la culture de cellules bactériennes est cultivée dans un milieu qui contient une source d'ions zinc et la culture de cellules bactériennes est cultivée à la température de 15 à 30 °C, de préférence 18 °C.

3. Procédé selon la revendication 2, **caractérisé en ce que** la source d'ions zinc est du chlorure de zinc à la concentration de 0,01 à 2 mM, de préférence 0,06 mM.

4. Utilisation de la composition telle que définie dans la revendication 1 dans les analyses d'amination/désamination et de méthylation/déméthylation d'ADN/ARN.
